# EUROPÄISCHE PATENTSCHRIFT

(11) **EP 0 384 266 B1**
(45) Veröffentlichungstag und Bekanntmachung des Hinweises auf die Patenterteilung: **20.07.1994**
(21) Anmeldenummer: 90102770.6
(22) Anmeldetag: 13.02.1990
(51) Int. Cl.: A61M 35/00, A61L 15/00

(54) **Therapeutisches System zur verzögerten und gesteuerten transdermalen oder transmucosalen Verabreichung von Wirkstoffen (II)**
Therapeutic system for the sustained and controlled transdermal or transmucosal delivery of active agents
Système thérapeutique pour l'administration transdermique ou transmucosale retardée et controlée d'agents actifs

(30) Priorität: 18.02.1989 DE 3905050
(43) Veröffentlichungstag der Anmeldung: 29.08.1990
(73) Patentinhaber: LTS Lohmann Therapie-Systeme GmbH & Co. KG, 56513 Neuwied (DE)
(72) Erfinder: Müller, Walter, Dr., Dipl.-Chem., D-5450 Neuwied 1 (DE)
(74) Vertreter: Flaccus, Rolf-Dieter, Dr.

(56) Entgegenhaltungen:
- EP-A- 0 197 504
- US-A- 4 781 924
- JOURNAL OF CONTROLLED RELEASE, Band 6, Dezember 1987, Elsevier Science Publishers B.V., Amsterdam, NL; C.D. EBERT et al.: "Development of a novel transdermal system design"

## Beschreibung

Die Erfindung betrifft ein therapeutisches System zur verzögerten und gesteuerten transdermalen oder transmucosalen Verabreichung von Wirkstoffen gemäß Oberbegriff des Anspruchs 1.

Unter therapeutischen Systemen sind arzneistoffhaltige Vorrichtungen bzw. Darreichungsformen zu verstehen, die einen Arzneistoff oder mehrere in vorausbestimmter Rate kontinuierlich über einen festgelegten Zeitraum an einen festgelegten Anwendungsort abgeben (siehe Heilmann, "Therapeutische Systeme", Enke Verlag, Stuttgart, 1984, Seite 24). Ein transdermales therapeutisches System gibt den Wirkstoff über die Haut ab und wird demgemäß auf der Haut, also topisch angewandt.

Ziel bei der Anwendung von Wirkstoffen sollte immer sein, den Wirkstoff in einer Menge zu verabreichen, die einerseits möglichst niedrig ist, andererseits aber mit größtmöglicher Sicherheit den gewünschten therapeutischen Effekt erwarten läßt. Zu diesem Zweck wurde eine ganze Reihe von sogenannten therapeutischen Systemen entwickelt, die den Wirkstoff auf eine durch die Systemparameter vorgegebene Weise gesteuert abgeben. Für eine ganze Reihe von systemisch wirkenden Wirkstoffen besteht nicht die Notwendigkeit oder der Wunsch, über den gesamten Zeitraum eines Tages gleichmäßig hohe Plasmaspiegel zu haben.

So ist es z.B. für einige Wirkstoffe von Vorteil, wenn die Wirkstoffspiegel während der Nachtruhe möglichst niedrig liegen und erst gegen Ende der Schlafperiode auf das therapeutisch notwendige Niveau angehoben werden.

Dabei ist besonders gedacht an Nitroverbindungen zur Angina pectoris Prophylaxe, da erstens Angina pectoris Anfälle in der Nacht selten und in den frühen Morgenstunden vergleichsweise häufig auftreten, und zweitens die bei diesen Nitroverbindungen mögliche Toleranzentwicklung schon durch eine mehrstündige Unterbrechung der Medikation vermieden werden kann. Aber auch für Nicotin, Appetitzügler, blutdruckbeeinflußende Mittel (β-Blocker, α-Sympathomimetika) oder Antiasthmatika (β-Sympathomimetika) wäre eine dermaßen auf den Bedarf angepaßte Dosierung wünschenswert.

Eine Arzneiform, die dieses leistet, muß die Wirkstoffabgabe an den Organismus nach der abendlichen Verabreichung um etwa 4-10 Stunden verzögern, so daß sich dann ohne weiteres Zutun des Patienten gegen Ende der Schlafphase therapeutisch notwendige Plasmaspiegel einstellen würden.

Besonders geeignet für diese doch recht lange Verzögerungszeit ist die transdermale oder transmucosale Verabreichung von Wirkstoffen. In der US-PS 4,655,766 wurde ein auf osmotischen Prinzipien beruhendes transdermales System, in der EP-A 0249475 ein auf Diffusionsprozessen basierendes System und in der EP-A 0249343 ein System beschrieben, welches erst durch die Zufuhr von Flüssigkeiten, wie z.B. der Hautfeuchtigkeit aktiviert wird.

Systeme gemäß EP-A 0249475 bestehen prinzipiell aus zwei getrennten Teilen, dem eigentlichen Wirkstoffreservoir und einem flächenförmigen wirkstofffreien Teil. Vor der Applikation wird die wirkstofffreie Schicht flächendeckend auf die Freigabeseite des Reservoirs aufgebracht, und dann das System mit der anderen Seite dieser wirkstofffreien Schicht auf die Haut geklebt. Zwischen den beiden Teilen des Systems besteht also ein Konzentrationsgradient bezüglich des Wirkstoffs. Dies hat nach den Fick'schen Diffusionsgesetzen zur Folge, daß der Wirkstoff in diese wirkstofffreie Schicht hineindiffundiert und nach einer durch die Systemparameter vorgegebenen Zeit auch die Haut erreicht. Die Verzögerungszeit entspricht also der Zeitspanne, die verstreicht vom Zeitpunkt der Vereinigung der vor Applikation getrennten Systemteile bis zu dem Zeitpunkt, ab dem der Wirkstoff mit einer für den therapeutischen Zweck ausreichenden Menge an die Haut abgegeben wird.

Ein Nachteil des Systemaufbaus ist, daß die wirkstofffreie Verzögerungsschicht einen zusätzlichen Diffusionswiderstand darstellt, der den maximalen Wirkstoffflux begrenzt.

Dieser Nachteil kann rein theoretisch vermieden werden durch einen Systemaufbau wie er in der EP-A 0249343 beschrieben wird. Dabei wird die Verzögerungsschicht durch eine Membran ersetzt, die nach einer gewissen Zeit nach der Aktivierung des Systems von "undurchlässig" auf "durchlässig" geschaltet wird. Die Aktivierung kann erfolgen durch die externe oder interne Zufuhr einer geeigneten Flüssigkeit. Diese Aktivierungsflüssigkeit kann z.B. auch die von der Haut abgegebene Feuchtigkeit sein.

Ein weiteres Beispiel eines derartigen Systemes wird in der US-A-4 781 924 beschrieben.

Ein Nachteil dieses Systems ist, daß es wohl sehr schwierig ist, die Durchlässigkeit einer Membran über die notwendige Größenordung zeitlich vorprogrammiert zu steuern. Dies gilt insbesondere, wenn als Aktivatorflüssigkeit die Hautfeuchtigkeit genommen wird, da dann individuelle Unterschiede aber auch so wenig kontrollierbare Parameter, wie Raumtemperatur oder Bekleidung eine große Rolle spielen.

Aufgabe der vorliegenden Erfindung war es nun, die Funktionssicherheit des in der EP-A 0249475 beschriebenen Systemaufbaus mit dem zumindest theoretisch möglichen hohen Wirkstoffflux des Systemaufbaus gemäß der EP-A 0249343 zu verbinden.

Die Lösung dieser Aufgabe gelang überraschenderweise durch Verzicht auf eine den Wirkstoffflux beeinflussende wirkstofffreie Verzögerungsschicht bzw. schaltbare Membran zwischen Wirkstoffreservoir und Haut. Der Wirkstoff darf allerdings zu dem Zeitpunkt der Applikation nicht in einer bioverfügbaren Form vorliegen. Bei der transdermalen oder transmucosalen Applikation heißt das, daß er entweder das System nicht verlassen oder nicht in die Haut penetrieren kann.

Im einzelnen wird diese Aufgabe gelöst entsprechend der Merkmale des kennzeichnenden Teils des Anspruchs 1

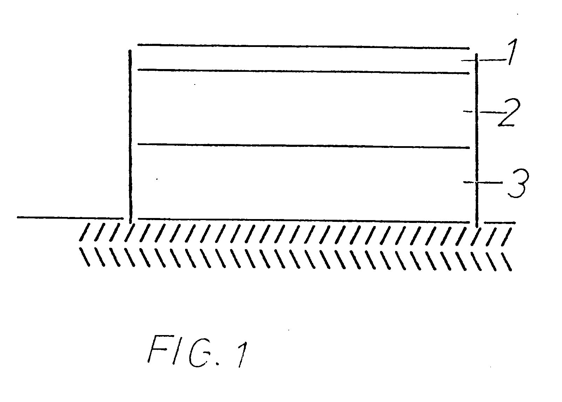

Fig. 1 zeigt eine Ausführungsform des erfindungsgemäßen Systems wie es nach Applikation und Aktivierung vorliegt.

Der sich im Wirkstoffreservoir befindende Wirkstoff wird durch den aus dem Hilfsstoffreservoir eindiffundierenden Hilfsstoff in seine bioverfügbare Form überführt.

Damit dies nicht schon während der Lagerzeit des Systems geschieht, muß die Hilfsstoffdiffusion bis frühestens zum Zeitpunkt der Applikation ausgeschlossen sein. Dies ist am einfachsten dadurch zu erreichen, daß beide Systeme getrennt geliefert werden und erst bei Applikation zu dem Gesamtsystem vereinigt werden, etwa indem zuerst das Wirkstoffreservoir und danach das Hilfsstoffreservoir mit der Rückschicht auf die gleiche Stelle appliziert werden. Es kann aber auch eine Vorrichtung, wie sie in der EP-A 0249475 beschrieben ist, benutzt werden.

Der Prozeß der Wirkstoffumwandlung setzt natürlich zuerst an der Grenzfläche der beiden Reservoire ein und pflanzt sich in Richtung der Freigabeseite fort. Der nun in seiner bioverfügbaren Form vorliegende Teil des Wirkstoffs wandert gemäß den Diffusionsgesetzen in Richtung Freigabeseite, aber auch zu einem gewissen Teil in das Hilfsstoffreservoir. Die Abgaberate an bioverfügbarem Wirkstoff aus dem System als Funktion der Zeit hängt in komplizerter Weise ab von dem Diffusionskoeffizienten des Hilfsstoffs im Wirkstoffreservoir, der Abgaberate des Hilfsstoffs aus dem Hilfsstoffreservoir, dem Diffusionskoeffizienten des Wirkstoffs in seiner bioverfügbaren Form im Wirkstoffreservoir, der Wirkstoffkonzentration, der Dicke des Wirkstoffreservoirs und der Lage des chemischen Gleichgewichts für die Überführung des Wirkstoffs aus einer nicht bioverfügbaren in seine bioverfügbare Form.

Trotz dieser komplizierten Zusammenhänge kann ein solches System, wenn es einmal optimiert ist, einfach und reproduzierbar gefertigt werden, da sein Funktionsprinzip auf einfachen Diffusionsvorgängen beruht.

In jedem Fall erreicht der Wirkstoff erst nach einer gewissen Zeit, eben der gewünschten Verzögerungszeit, sein Zielorgan, die Haut bzw. Schleimhaut.

Zur Erreichung dieser Verzögerungszeit wird dabei keine den Wirkstoffflux begrenzende wirkstofffreie Schicht zwischen Wirkstoffreservoir und Abgabefläche des Systems benötigt.

Systeme im Sinne dieser Erfindung bieten prinzipiell zwei Möglichkeiten, den Wirkstoff in einer nicht bioverfügbaren Form zu enthalten:
a. der Wirkstoff ist immobilisiert
b. der Wirkstoff liegt als Salz vor
Immobilisiert ist der Wirkstoff immer dann, wenn er an ein selbst nicht zur Diffusion befähigtes Polymer gebunden ist. Dies ist der Fall, wenn der Wirkstoff eine Säure oder eine Base ist und als Salz einer polymeren Polybase bzw. Polysäure vorliegt.

Die Herstellung solcher Salze ist eine einfache Säure-Base Reaktion und geschieht zwangsläufig bei dem Zusammengeben beider Komponenten in einem geeigneten Lösemittel gemäß untenstehenden Gleichungen. 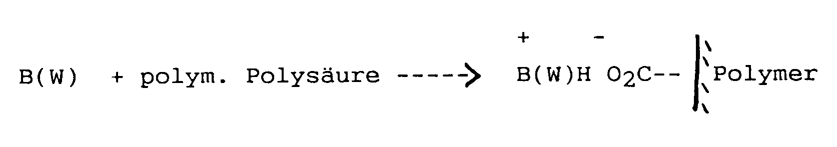
B(W) = Wirkstoffbase 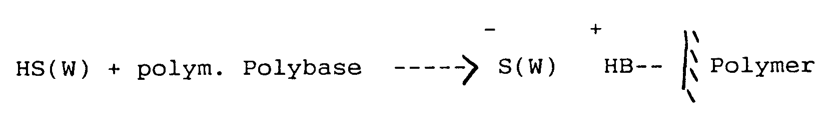
HS(W) = Wirkstoffsäure
In Umkehrung dieser Reaktion setzt der Hilfsstoff, der je nach vorliegendem Fall selbst Säure- oder Basecharakter hat, die ungebundene Wirkstoffbase bzw. Wirkstoffsäure frei. 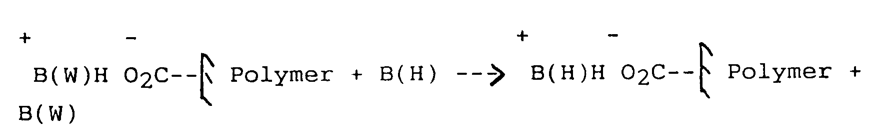
B(H) = Hilfsstoffbase 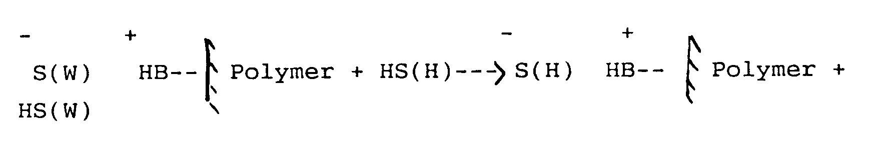
HS(H) = Hilfsstoffsäure
Geeignete polymere Polysäuren bzw. Polybasen sind Ionentauscher auf Kunstharzbasis in ihrer sauren bzw. basischen Form, aber auch Acrylharze mit sauren oder basischen funktionellen Gruppen. Besonders geeignet unter den Acrylharzen sind das basische Eudragit E 100 und das saure Eudragit L 100 bzw. S 100 der Fa. Röhm-Pharma.

Aber auch wenn der Wirkstoff in einer zur Diffusion befähigten Form vorliegt, kann er durch geeignete Maßnahmen nicht bioverfügbar gemacht werden.

Speziell bei transdermaler Applikation von Wirkstoffen ist die lipophile Barriere des Stratum Corneum zu überwinden. Dies ist naturgemäß besonders schwierig für Substanzen, die polarer oder ionischer Natur sind. Da die meisten Wirkstoffe entweder selbst Basen oder Säuren sind, ist es ohne weiteres möglich, sie durch Salzbildung so hydrophil zu machen, daß sie als nunmehr ionische Verbindungen zwar zur Diffusion im System befähigt sind, aber nicht mehr das Stratum Corneum passieren können und damit also nicht mehr bioverfügbar sind.

Auch hier setzt der Hilfsstoff analog zu obigen Gleichungen wieder die Wirkstoffsäure bzw. Wirkstoffbase frei, die als nunmehr ungeladene Moleküle deutlich weniger hydrophil sind und damit leichter das Stratum Corneum passieren können.

Als Hilfsstoffsäuren eignen sich prinzipiell alle physiologisch unbedenklichen Säuren, insbesondere aber die niedrigen einwertigen aliphatischen Carbonsäuren und deren Derivate. Beispiele für geeignete Säuren sind: Essigsäure, Propionsäure, Bernsteinsäure, Benzoesäure. Als Hilfsstoffbasen kommen in Frage alle physiologisch unbedenklichen Basen, insbesondere aber basische Stickstoffverbindungen, wie z.B. Amine, Ethanolamine, Glycinderivate oder andere basische Aminosäurederivate.

Eine andere Möglichkeit, eine verzögerte Wirkstoffaufnahme zu erreichen beruht darauf, die schlechte Hautgängigkeit von manchen Wirkstoffen bzw. Wirkstoffderivaten und deren Beeinflussungsmöglichkeit durch sogenannte Penetrationsbeschleuniger auszunutzen. In diesem Fall wird der Wirkstoff ohne Penetrationsbeschleuniger, der sich nun als Hilfsstoff im Hilfsstoffreservoir befindet, in einer weit unter der notwendigen oder Wirkung zeigenden Dosis aufgenommen. Erst wenn der Penetrationsbeschleuniger das Wirkstoffreservoir passiert hat und selbst in die Haut penetriert ist, steigert er die Wirkstoffaufnahme auf das therapeutisch notwendige Niveau. Das Wirkstoffreservoir fungiert hier also gewissermaßen als Verzögerungsschicht für den Penetrationsbeschleuniger.

In der Literatur sind eine Vielzahl von Substanzen bzw. Substanzgruppen beschrieben, die die Wirkstoffaufnahme recht drastisch - manchmal um bis zu zwei Größenordnungen - erhöhen. Beispielhaft sei genannt Azone der Fa. Nelson Research & Development Company, EP-A 0095169.

Als Materialien für die Reservoire bzw. für die Rückschicht und die wieder ablösbare Schutzschicht können alle Materialien verwendet werden, die für die Herstellung von transdermalen und transmucosalen Systemen üblich und dem Fachmann hinreichend bekannt sind.

Als Materialien für die Rückschicht geeignet sind z.B. Folien aus Polyester, PVC, Polyamid, Polyethylen oder Polypropylen. Gebräuchlich sind auch Verbundfolien aus diesen Materialien, wobei oftmals eine zusätzliche Aluminiumschicht für die Undurchlässigkeit für Wirkstoffe sorgt.
Als Materialien für die wieder ablösbare Schutzschicht sind prinzipiell die gleichen Materialien wie für die Rückschicht verwendbar, die jedoch zusätzlich abhäsiv ausgerüstet sein müssen.

Als Grundmaterialien für die Reservoire seien beispielhaft genannt: Polyisobutylene, Styrol-Isopren-Styrol Blockpolymerisate, Polysiloxane, Polymethacrylate, Polyurethane, Polyester, Polyamide und Copolymerisate des Ethylens mit z.B. Vinylacetat oder Acrylsäurederivaten.

Die Reservoire selbst können beliebig kompliziert aufgebaut sein. Insbesondere können zusätzliche, die Abgabe des Hilfsstoffs an das Wirkstoffreservoir und/oder die Abgabe des Wirkstoffs in seiner bioverfügbaren Form aus dem System steuernde, Membranen sinnvoll sein, um das gewünschte Freisetzungsprofil zu erreichen.

In Figur 1, welche die Erfindung erläutert, bedeuten
1) die Rückschicht
2) das Hilfsstoffreservoir und
3) das Wirkstoffreservoir
Als Wirkstoffe sind beispielsweise geeignet Nicotin, Appetitzügler wie Norpseudoephedrin, Amfepramon, Mefenorex, Propylhexedrin, Fenfluramin oder Mazindol, Betablocker wie Alprenolol, Oxprenolol, Penbutolol, Bupranolol, Metoprolol, Betaxolol, Atenolol, Acebutolol, Metipranolol, Propranolol, Nadolol, Pindolol, Mepindolol, Carteolol, Carazolol, Timolol oder Sotalol, Wirkstoffe aus der Gruppe der α-Sympathomimetika wie Norfenetrin, Octapamin, Oxedrin, Metaraminol, Midodrin oder Oxilofrin sowie Wirkstoffe aus der Gruppe der β-Sympathmimetika wie Salbutamol, Terbutalin, Fenoterol, Clenbuterol, Reproterol, Hexoprenalin, Bamethan oder Isoxsuprin.

## Patentansprüche (Patentansprüche für folgende(n) Vertragsstaat(en): AT, BE, CH, DE, DK, FR, GB, IT, LI, LU, NL, SE)

1. Therapeutisches System zur verzögerten und gesteuerten transdermalen oder transmucosalen Verabreichung von Wirkstoffen aus einer undurchlässigen Rückschicht, einem Hilfsstoffreservoir, einem Wirkstoffreservoir und einer wieder ablösbaren Schutzschicht, dadurch gekennzeichnet, daß
- der im Wirkstoffreservoir vorhandene saure oder basische Wirkstoff an ein nicht zur Diffusion befähigtes Polymer gebunden ist und damit als Salz einer polymeren Polybase bzw. Polysäure in immobilisierter Form vorliegt,
- daß der im Hilfsstoffreservoir vorhandene Hilfsstoff zur Überführung des Wirkstoffsalzes bei Kontakt des Hilfsstoffreservoirs mit dem Wirkstoffreservoir in eine diffusionsfähige Form sauren bzw. basischen Character besitzt,
- und daß das Wirkstoffreservoir und das Hilfsstoffreservoir zumindest bis zum Zeitpunkt der Applikation des Systems keinen Kontakt miteinander haben.

2. Therapeutisches System gemäß Anspruch 1, dadurch gekennzeichnet, daß das Wirkstoffreservoir über eine Freigabefläche direkt oder über einen selbstklebenden Aufstrich Kontakt mit der Applikationsstelle hat.

3. Therapeutisches System gemäß Anspruch 1 und 2, dadurch gekennzeichnet, daß Hilfsstoff- und Wirkstoffreservoir zur Herstellung des die Diffusion des Hilfsstoffs in das Wirkstoffreservoir ermöglichenden Kontakts aufeinanderlaminiert werden.

4. Therapeutisches System gemäß Anspruch 1 und 2, dadurch gekennzeichnet, daß die wirkstofffreie Schicht einen sauer eingestellten Puffer enthält.

5. Therapeutisches System gemäß Anspruch 1, dadurch gekennzeichnet, daß der Wirkstoff eine Säure ist und die wirkstofffreie Schicht eine Base enthält.

6. Therapeutisches System gemäß Anspruch 1 oder 2, dadurch gekennzeichnet, daß der Hilfsstoff und der Wirkstoff Basen sind und der Wirkstoff an eine polymere Polysäure gebunden ist.

7. Therapeutisches System gemäß Anspruch 1 und 2, dadurch gekennzeichnet, daß der Wirkstoff und der Hilfsstoff Säuren sind und der Wirkstoff an eine polymere Polybase gebunden ist.

8. Therapeutisches System gemäß Anspruch 1 und 2, dadurch gekennzeichnet, daß der Wirkstoff als nicht polymeres Salz vorliegt und der Hilfsstoff den Wirkstoff in die freie Base oder Säure überführt.

9. Therapeutisches System gemäß Anspruch 8, dadurch gekennzeichnet, daß der basische Wirkstoff als Ammoniumsalz vorliegt und der Hilfsstoff eine Base ist.

10. Therapeutisches System gemäß Anspruch 1 und 2, dadurch gekennzeichnet, daß der Hilfsstoff ein Penetrationsbeschleuniger ist.

11. Therapeutisches System gemäß Anspruch 1 und 2, dadurch gekennzeichnet, daß der Wirkstoff Nicotin ist.

12. Therapeutisches System gemäß Anspruch 1 und 2, dadurch gekennzeichnet, daß der Wirkstoff ein Appetitzügler ist.

13. Therapeutisches System gemäß Anspruch 12, dadurch gekennzeichnet, daß der Appetitzügler Norpseudoephedrin, Amfepramon, Mefenorex, Propylhexedrin, Fenfluramin oder Mazindol ist.

14. Therapeutisches System gemäß Anspruch 1 und 2, dadurch gekennzeichnet, daß der Wirkstoff ein Betablocker ist.

15. Therapeutisches System gemäß Anspruch 14, dadurch gekennzeichnet, daß der Betablocker Alprenolol, Oxprenolol, Penbutolol, Bupranolol, Metoprolol, Betaxolol, Atenolol, Acebutolol, Metipranolol, Propranolol, Nadolol, Pindolol, Mepindolol, Carteolol, Carazolol, Timolol oder Sotalol ist.

16. Therapeutisches System gemäß Anspruch 1 und 2, dadurch gekennzeichnet, daß der Wirkstoff zur Gruppe der α -Sympathomimetika gehört.

17. Therapeutisches System gemäß Anspruch 16, dadurch gekennzeichnet, daß der Wirkstoff Norfenetrin, Octopamin, Oxedrin, Metaraminol, Midodrin oder Oxilofrin ist.

18. Therapeutisches System gemäß Anspruch 1 und 2, dadurch gekennzeichnet, daß der Wirkstoff zur Gruppe der β-Sympathomimetika gehört.

19. Therapeutisches System gemäß Anspruch 18, dadurch gekennzeichnet, daß der Wirkstoff Salbutamol, Terbutalin, Fenoterol, Clenbuterol, Reproterol, Hexoprenalin, Bamethan oder Isoxsuprin ist.

## Patentansprüche (Patentansprüche für folgende(n) Vertragsstaat(en): GR, ES)

1. Verfahren zur Herstellung eines therapeutischen Systems zur verzögerten und gesteuerten transdermalen oder transmucosalen Verabreichung von Wirkstoffen aus einer undurchlässigen Rückschicht, einem Hilfsstoffreservoir, einem Wirkstoffreservoir und einer wieder ablösbaren Schutzschicht, dadurch gekennzeichnet, daß
- der im Wirkstoffreservoir vorhandene saure oder basische Wirkstoff an ein nicht zur Diffusion befähigtes Polymer gebunden ist und damit als Salz einer polymeren Polybase bzw. Polysäure in immobilisierter Form vorliegt,
- daß der im Hilfsstoffreservoir vorhandene Hilfsstoff zur Überführung des Wirkstoffsalzes bei Kontakt des Hilfsstoffreservoirs mit dem Wirkstoffreservoir in eine diffusionsfähige Form sauren bzw. basischen Character besitzt,
- und daß das Wirkstoffreservoir und das Hilfsstoffreservoir zumindest bis zum Zeitpunkt der Applikation des Systems keinen Kontakt miteinander haben.

2. Verfahren nach Anspruch 1, dadurch gekennzeichnet, daß ein Wirkstoffreservoir verwendet wird, welches über eine Freigabefläche direkt oder über einen selbstklebenden Aufstrich Kontakt mit der Applikationsstelle hat.

3. Verfahren nach Anspruch 1 und 2, dadurch gekennzeichnet, daß Hilfsstoff- und Wirkstoffreservoir zur Herstellung des die Diffusion des Hilfsstoffs in das Wirkstoffreservoir ermöglichenden Kontakts aufeinanderlaminiert werden.

4. Verfahren nach Anspruch 1 und 2, dadurch gekennzeichnet, daß ein wirkstofffreies Hilfstoffreservoir verwendet wird, welches einen sauer eingestellten Puffer enthält.

5. Verfahren nach Anspruch 1 oder 2, dadurch gekennzeichnet, daß als Hilfs- und Wirkstoff Basen und der Wirkstoff an eine polymere Polysäure gebunden verwendet werden.

6. Verfahren nach System Anspruch 1 und 2, dadurch gekennzeichnet, daß als Wirkstoff und Hilfsstoff Säuren und der Wirkstoff an eine polymere Polybase gebunden verwendet werden.

7. Verfahren nach Anspruch 1 und 2, dadurch gekennzeichnet, daß als Wirkstoff ein nicht polymeres Salz verwendet wird, wobei der Hilfsstoff den Wirkstoff in die freie Base oder Säure überführt.

8. Verfahren nach Anspruch 7, dadurch gekennzeichnet, daß als basische Wirkstoff ein Ammoniumsalz und als Hilfsstoff eine Base verwendet werden.

9. Verfahren nach Anspruch 1 und 2, dadurch gekennzeichnet, daß als Hilfsstoff ein Penetrationsbeschleuniger verwendet wird.

10. Verfahren nach Anspruch 1 und 2, dadurch gekennzeichnet, daß als Wirkstoff Nicotin verwendet wird.

11. Verfahren nach Anspruch 1 und 2, dadurch gekennzeichnet, daß als Wirkstoff ein Appetitzügler verwendet wird.

12. Verfahren nach Anspruch 11, dadurch gekennzeichnet, daß als Appetitzügler Norpseudoephedrin, Amfepramon, Mefenorex, Propylhexedrin, Fenfluramin oder Mazindol verwendet werden.

13. Verfahren nach Anspruch 1 und 2, dadurch gekennzeichnet, daß als Wirkstoff ein Betablocker verwendet wird.

14. Verfahren nach Anspruch 13, dadurch gekennzeichnet, daß als Betablocker Alprenolol, Oxprenolol, Penbutolol, Bupranolol, Metoprolol, Betaxolol, Atenolol, Acebutolol, Metipranolol, Propranolol, Nadolol, Pindolol, Mepindolol, Carteolol, Carazolol, Timolol oder Sotalol verwendet werden.

15. Verfahren nach Anspruch 1 und 2, dadurch gekennzeichnet, daß als Wirkstoffe α-Sympathomimetika verwendet werden.

16. Verfahren nach Anspruch 15, dadurch gekennzeichnet, daß als Wirkstoff Norfenetrin, Octopamin, Oxedrin, Metaraminol, Midodrin oder Oxilofrin verwendet werden.

17. Verfahren nach gemäß Anspruch 1 und 2, dadurch gekennzeichnet, daß als Wirkstoffe β-Sympathomimetika verwendet werden.

18. Verfahren nach Anspruch 17, dadurch gekennzeichnet, daß als Wirkstoff Salbutamol, Terbutalin, Fenoterol, Clenbuterol, Reproterol, Hexoprenalin, Bamethan oder Isoxsuprin verwendet werden.

## Claims (Claims for the following Contracting State(s): AT, BE, CH, DE, DK, FR, GB, IT, LI, LU, NL, SE)

1. Therapeutic system for the retarded and controlled transdermal or transmucous administration of active substances, consisting of an impermeable backing layer, an auxiliary agent reservoir, an active substance reservoir and a removable protective layer, characterized in that
- the acid or basic active substance present in the active substance reservoir is bonded to a polymer not capable of diffusion and is thus present as a salt of a polymeric polybase or polyacid in immobilized form,
- in order to convert the active substance salt upon contact of the auxiliary agent reservoir with the active substance reservoir, into a form which is diffusible, the auxiliary agent present in the auxiliary agent reservoir is of acid or basic character, respectively,
- and that the active substance reservoir and the auxiliary substance reservoir do contact each other at least until the moment of application of the system

2. The therapeutic system according to claim 1, characterized in that the active substance reservoir contacts the site of application either directly via a releasing surface or via a self-adhesive coat.

3. The therapeutic system according to claims 1 and 2, characterized in that auxiliary agent reservoir and active substance reservoir are laminated with one another in order to create the contact allowing the diffusion of the auxiliary agent into the active substance reservoir.

4. The therapeutic system according to claims 1 and 2, characterized in that the drug-free layer comprises an acidified buffer.

5. The therapeutic system according to claim 1, characterized in that the active substance is an acid and the active substance-free layer comprises a base.

6. The therapeutic system according to claims 1 or 2, characterized in that the auxiliary agent and the active substance are bases and the active substance is bonded to a polymeric polyacid.

7. The therapeutic system according to claims 1 and 2, characterized in that the active substance and the auxiliary agent are acids and the active substance is bonded to a polymeric polybase.

8. The therapeutic system according to claims 1 and 2, characterized in that the active substance is present as non-polymeric salt and the auxilary agent converts the active substance into the free base or acid.

9. The therapeutic system according to claim 8, characterized in that the basic active substance is present as ammonium salt and the auxiliary agent is a base.

10. The therapeutic system according to claims 1 and 2, characterized in that the auxiliary agent is a penetration accelerator.

11. The therapeutic system according to claims 1 and 2, characterized in that the active substance is nicotine.

12. The therapeutic system according to claims 1 and 2, characterized in that the active substance is an appetite-suppressing agent.

13. The therapeutic system according to claim 12, characterized in that the appetite-suppressing agent is norpseudoephedrine, amfepramone, mefenorex, propylhexedrine, fenfluramine, or mazindol.

14. The therapeutic system according to claims 1 and 2, characterized in that the active substance is a beta-blocker.

15. The therapeutic system according to claim 14, characterized in that the beta-blocker is alprenolol, oxprenolol, penbutolol, bupranolol, metoprolol, betaxolol, atenolol, acebutolol, metipranolol, propranolol, nadolol, pindolol, mepindolol, carteolol, carazolol, timolol, or sotalol.

16. The therapeutic system according to claims 1 and 2, characterized in that the active substance belongs to the group of α-sympathomimetics.

17. The therapeutic system according to claim 16, characterized in that the active substance is norfenetrine, octapamine, oxedrine, metaraminol, midodrine, or oxilofrine.

18. The therapeutic system according to claims 1 and 2, characterized in that the active substance belongs to the group of β-sympathomimetics.

19. The therapeutic system according to claim 18, characterized in that the active substance is salbutamol, terbutaline, fenoterol, clenbuterol, reproterol, hexoprenaline, bamethan, or isoxsuprine.

## Claims (Claims for the following Contracting State(s): GR, ES)

1. Process for the production of a therapeutic system for the retarded and controlled transdermal or transmucous administration of active substances, consisting of an impermeable backing layer, an auxiliary agent reservoir, an active substance reservoir and a removable protective layer, characterized in that
- the acid or basic active substance present in the active substance reservoir is bonded to a polymer not capable of diffusion and is thus present as a salt of a polymeric polybase or polyacid in immobilized form,
- in order to convert the active substance salt upon contact of the auxiliary agent reservoir with the active substance reservoir, into a form which is diffusible, the auxiliary agent present in the auxiliary agent reservoir is of acid or basic character, respectively,
- and that the active substance reservoir and the auxiliary substance reservoir do contact each other at least until the moment of application of the system

2. The process according to claim 1, characterized in that an active substance reservoir is employed which is in contact with the site of application either directly via a releasing surface or via a self-adhesive coat.

3. The process according to claims 1 and 2, characterized in that auxiliary agent reservoir and active substance reservoir are laminated with one another in order to create the contact allowing the diffusion of the auxiliary agent into the active substance reservoir.

4. The process according to claims 1 and 2, characterized in that a drug-free layer is used comprising an acidified buffer.

5. The process according to claims 1 or 2, characterized in that as auxiliary agent and active substance, bases are used and that an active substance is used which is bonded to a polymeric polyacid.

6. The process according to claims 1 and 2, characterized in that as active substance and auxiliary agent, acids are used and that an active substance is used which is bonded to a polymeric polybase.

7. The process according to claims 1 and 2, characterized in that as active substance a non-polymeric salt is used, whereby the auxilary agent converts the active substance into the free base or acid.

8. The process according to claim 7, characterized in that as basic active substance an ammonium salt, and as auxiliary agent a base is used.

9. The process according to claims 1 and 2, characterized in that as auxiliary agent a penetration accelerator is used.

10. The process according to claims 1 and 2, characterized in that nicotine is used as active substance.

11. The process according to claims 1 and 2, characterized in that as active substance an appetite-suppressing agent is used.

12. The process according to claim 11, characterized in that as appetite-suppressing agent norpseudoephedrine, amfepramone, mefenorex, propylhexedrine, fenfluramine, or mazindol are used.

13. The process according to claims 1 and 2, characterized in that as active substance a beta blocker is employed.

14. The process according to claim 13, characterized in that as beta blocker alprenolol, oxprenolol, penbutolol, bupranolol, metoprolol, betaxolol, atenolol, acebutolol, metipranolol, propranolol, nadolol, pindolol, mepindolol, carteolol, carazolol, timolol or sotalol are used.

15. The process according to claims 1 and 2, characterized in that as active substances α-sympathomimetics are employed.

16. The process according to claim 15, characterized in that as active substance norfenetrine, octapamine, oxedrine, metaraminol, midodrine, or oxilofrine are used.

17. The process according to claims 1 and 2, characterized in that as active substances β-sympathomimetics are employed.

18. The therapeutic system according to claim 17, characterized in that as active substance salbutamol, terbutaline, fenoterol, clenbuterol, reproterol, hexoprenaline, bamethan, or isoxsuprine are used.

## Revendications (Revendications pour l'(les) Etat(s) contractant(s) suivant(s): AT, BE, CH, DE, DK, FR, GB, IT, LI, LU, NL, SE)

1. Système thérapeutique pour l'administration transdermique ou transmucosale, retardée et réglée, de principes actifs, constitué d'une couche dorsale imperméable, d'un réservoir d'adjuvant, d'un réservoir de principe actif et d'une couche de protection amovible, caractérisé en ce que
- le principe actif acide ou basique, présent dans le réservoir de principe actif, est lié à un polymère susceptible de diffusion et se présente, de ce fait, sous une forme immobilisée en tant que sel d'une polybase ou d'un polyacide polymérique,
- l'adjuvant présent dans le réservoir d'adjuvant pour la conversion du sel du principe actif en une forme susceptible de diffusion par contact du réservoir d'adjuvant avec le réservoir de principe actif, possède un caractère acide ou basique et
- le réservoir de principe actif et le réservoir d' adjuvant n'ont aucun contact mutuel au moins jusqu'au moment de l'application du système.

2. Système thérapeutique suivant la revendication 1, caractérisé en ce que le réservoir de principe actif a un contact, par l'intermédiaire d'une surface de libération, direct ou à l'aide d'une couche autocollante, avec l'endroit d'application.

3. Système thérapeutique suivant les revendications 1 et 2, caractérisé en ce que le réservoir d'adjuvant et le réservoir de principe actif sont lamifiés l'un sur l'autre en vue de la réalisation du contact permettant la diffusion de l'adjuvant dans le réservoir de principe actif.

4. Système thérapeutique suivant les revendications 1 et 2, caractérisé en ce que la couche dépourvue de principe actif contient un tampon réglé du côté acide.

5. Système thérapeutique suivant la revendication 1, caractérisé en ce que le principe actif est un acide et la couche dépourvue de principe actif contient une base.

6. Système thérapeutique suivant la revendication 1 ou 2, caractérisé en ce que l'adjuvant et le principe actif sont des bases et le principe actif est lié à un polyacide polymérique.

7. Système thérapeutique suivant les revendications 1 et 2, caractérisé en ce que le principe actif et l'adjuvant sont des acides et le principe actif est lié à une polybase polymérique.

8. Système thérapeutique suivant les revendications 1 et 2, caractérisé en ce que le principe actif se présente sous forme d'un sel non polymérique et l'adjuvant convertit le principe actif en la base libre ou l'acide libre.

9. Système thérapeutique suivant la revendication 8, caractérisé en ce que le principe actif basique se présente sous forme de sel d'ammonium et l'adjuvant est une base.

10. Système thérapeutique suivant les revendications 1 et 2, caractérisé en ce que l'adjuvant est un accélérateur de pénétration.

11. Système thérapeutique suivant les revendications 1 et 2, caractérisé en ce que le principe actif est la nicotine.

12. Système thérapeutique suivant les revendications 1 et 2, caractérisé en ce que le principe actif est un produit anorexigène.

13. Système thérapeutique suivant la revendication 12, caractérisé en ce que le produit anorexigène est la norpseudoéphédrine, l'amfépramon, le méfénorex, la propylhexédrine, la fenfluramine ou le mazindole.

14. Système thérapeutique suivant les revendications 1 et 2, caractérisé en ce que le principe actif est un β-bloquant.

15. Système thérapeutique suivant la revendication 14, caractérisé en ce que le β-bloquant est l'un des composés qui suivent : alprénolol, oxprénolol, penbutolol, bupranolol, métoprolol, bêtaxolol, aténolol, acébutolol, métipranolol, propanolol, nadolol, pindolol, mépindolol, cartéolol, carazolol, timolol ou sotalol.

16. Système thérapeutique suivant les revendications 1 et 2, caractérisé en ce que le principe actif appartient au groupe des α-sympathicomimétiques.

17. Système thérapeutique suivant la revendication 16, caractérisé en ce que le principe actif est la norfénétrine, l'octopamine, l'oxédrine, le métaraminol, la midodrine ou l'oxilofrine.

18. Système thérapeutique suivant les revendications 1 et 2, caractérisé en ce que le principe actif appartient au groupe des β-sympathicomimétiques.

19. Système thérapeutique suivant la revendication 18, caractérisé en ce que le principe actif est le salbutamol, la terbutaline, le fénotérol, le clenbutérol, le réprotérol, l'hexoprénaline, le baméthane ou l'isoxsuprine.

## Revendications (Revendications pour l'(les) Etat(s) contractant(s) suivant(s): GR, ES)

1. Procédé de préparation d'un système thérapeutique pour l'administration transdermique ou transmucosale, retardée et réglée, de principes actifs, constitué d'une couche dorsale imperméable, d'un réservoir d'adjuvant, d'un réservoir de principe actif et d'une couche de protection amovible, caractérisé en ce que
- le principe actif acide ou basique, présent dans le réservoir de principe actif, est lié à un polymère susceptible de diffusion et se présente, de ce fait, sous une forme immobilisée en tant que sel d'une polybase ou d'un polyacide polymérique,
- l'adjuvant présent dans le réservoir d'adjuvant pour la conversion du sel du principe actif en une forme susceptible de diffusion par contact du réservoir d'adjuvant avec le réservoir de principe actif, possède un caractère acide ou basique et
- le réservoir de principe actif et le réservoir d' adjuvant n'ont aucun contact mutuel au moins jusqu'au moment de l'application du système.

2. Procédé suivant la revendication 1, caractérisé en ce que l'on utilise un réservoir de principe actif qui a un contact, par l'intermédiaire d'une surface de libération, direct ou à l'aide d'une couche autocollante, avec l'endroit d'application.

3. Procédé suivant les revendications 1 et 2, caractérisé en ce que le réservoir d'adjuvant et le réservoir de principe actif sont lamifiés l'un sur l'autre en vue de la réalisation du contact permettant la diffusion de l'adjuvant dans le réservoir de principe actif.

4. Procédé suivant les revendications 1 et 2, caractérisé en ce que l'on utilise un réservoir d'adjuvant dépourvue de principe actif, qui contient un tampon réglé du côté acide.

5. Procédé suivant les revendications 1 ou 2, caractérisé en ce que l'on utilise des bases à titre de principe actif et d'adjuvant et le principe actif est lié à un polyacide polymérique.

6. Procédé suivant les revendications 1 ou 2, caractérisé en ce que l'on utilise des acides à titre de principe actif et d'adjuvant et le principe actif est lié à une polyacide polymérique.

7. Procédé suivant les revendications 1 et 2, caractérisé en ce que l'on utilise un sel non polymérique à titre de principe actif, où l'adjuvant convertit le principe actif en la base libre ou l'acide libre.

8. Procédé suivant la revendication 7, caractérisé en ce que l'on utilise un sel d'ammoniun à titre de principe actif basique et une base à titre d'adjuvant.

9. Procédé suivant les revendications 1 et 2, caractérisé en ce que l'on utilise un accélérateur de pénétration à titre d'adjuvant.

10. Procédé suivant les revendications 1 et 2, caractérisé en ce que l'on utilise la nicotine à titre de principe actif.

11. Procédé suivant les revendications 1 et 2, caractérisé en ce que l'on utilise un produit anorexique à titre de principe actif.

12. Procédé suivant la revendication 11, caractérisé en ce que l'on utilise, à titre de produit anorexique, la norpseudoéphédrine, l'amfépramon, le méfénorex, la propylhexédrine, la fenfluramine ou le mazindole.

13. Procédé suivant les revendications 1 et 2, caractérisé en ce que l'on utilise un β-bloquant à titre de principe actif.

14. Procédé suivant la revendication 13, caractérisé en ce que, à titre de β-bloquant, on utilise l'une des substances qui suivent : alprénolol, oxprénolol, penbutolol, bupranolol, métoprolol, bêtaxolol, aténolol, acébutolol, métipranolol, propanolol, nadolol, pindolol, mépindolol, cartéolol, carazolol, timolol ou sotalol.

15. Procedé suivant les revendications 1 et 2, caractérisé en ce que l'on utilise des α-sympathicomimétiques à titre de principes actifs.

16. Procédé suivant la revendication 15, caractérisé en ce que, à titre de principe actif, on utilise la norfénétrine, l'octopamine, l'oxédrine, le métaraminol, la midodrine ou l'oxilofrine.

17. Procédé suivant les revendications 1 et 2, caractérisé en ce que l'on utilise des β-sympathicomimétiques à titre de principes actifs.

18. Procédé suivant la revendication 17, caractérisé en ce que, à titre de principe actif, on utilise le salbutamol, la terbutaline, le fénotérol, le clenbutérol, le réprotérol, l'hexoprénaline, le baméthane ou l'isoxsuprine.
